# FASCICULE DE BREVET EUROPEEN

(11) **EP 4 615 422 B1**
(45) Date de publication et mention de la délivrance du brevet: **12.08.2026**
(21) Numéro de dépôt: 23802284.2
(22) Date de dépôt: 09.11.2023
(51) Int. Cl.: A61K 9/19, A61K 9/51, A61K 31/375, A61K 47/54, A61P 7/06, A61K 9/00, A61K 9/14, A61K 9/16, A23L 33/10, A61K 47/55, A23L 33/15, A23L 33/175, A61K 33/26

(54) **NANOCLUSTERS DE FER, LEURS PROCÉDÉS D'OBTENTION ET LEURS UTILISATIONS POUR LUTTER CONTRE LES CARENCES EN FER**
EISENNANOCLUSTER, VERFAHREN ZUR HERSTELLUNG DAVON UND VERWENDUNGEN DAVON ZUR BEKÄMPFUNG VON EISENMANGELERSCHEINUNGEN
IRON NANOCLUSTERS, METHODS FOR OBTAINING THEM AND USES THEREOF FOR CONTROLLING IRON DEFICIENCIES

(30) Priorité: 10.11.2022 EP 22315281
(43) Date de publication de la demande: 17.09.2025
(73) Titulaire: Université de Lorraine, 54000 Nancy (FR); Centre Hospitalier Régional et Universitaire de Nancy, 54000 Nancy (FR); Institut National de la Santé et de la Recherche Médicale, 75013 Paris (FR)
(72) Inventeur: BOUDIER, Ariane, 54130 SAINT-MAX (FR); CLAROT, Igor, 54360 EINVAUX (FR); FEILLET, François, 54110 DOMBASLE SUR MEURTHE (FR)
(74) Mandataire: Plasseraud IP
(86) Numéro de dépôt international: PCT/EP2023/081345
(87) Numéro de publication internationale: WO 2024/100213

(56) Documents cités:
- WO-A1-2010/034319
- CN-A- 105 965 028
- US-A1- 2012 093 898
- US-A1- 2016 022 733

## Description

### Domaine technique

La présente invention relève du domaine de la chimie, et plus particulièrement de la chimie pharmaceutique. L'invention concerne des nanoclusters de fer, leurs procédés d'obtention et leurs utilisations pour lutter contre les carences en fer, et plus particulièrement pour leurs utilisations dans la prévention et/ou le traitement de pathologies générant un déficit en fer.

### Technique antérieure

La carence en fer, encore appelée carence martiale ou déficit martial, désigne le manque de fer dans l'organisme. Le fer est un métal indispensable au fonctionnement de l'organisme et dont l'origine est uniquement alimentaire. La carence en fer est l'une des carences minérales les plus répandues dans le monde : ainsi, plus d'1,5 milliard d'êtres humain souffrent d'une carence en fer dans le monde.

Le fer est présent dans toutes les cellules du corps humain et est responsable de nombreuses fonctions vitales dont le transport de l'oxygène via sa présence dans l'hémoglobine. Une carence en fer peut interférer avec ces fonctions vitales dont le premier signe est une anémie microcytaire pouvant aller jusqu'au décès de l'individu dans les cas extrêmes.

La majorité du fer présent dans l'organisme (70%) se trouve sous forme héminique, c'est-à-dire associé à l'hémoglobine dans le sang (65%) ou à la myoglobine dans les muscles (5%). Le fer restant se trouve sous forme non héminique (ferritine, transferrine ...).

L'absorption du fer est un mécanisme finement régulé. Lorsque les réserves de fer de l'organisme diminuent, son taux d'absorption augmente, et, inversement, lorsque les réserves en fer sont élevées, l'absorption baisse, ce qui évite l'accumulation excessive de fer dans l'organisme (une accumulation de fer entraîne une hémochromatose).

L'alimentation apporte en moyenne 10 à 15 mg de fer par jour. Seulement 1 à 2 mg sont absorbés dans la partie supérieure de l'intestin grêle. L'absorption du fer dépend de sa nature, de la qualité du repas et de l'état des réserves d'un individu. 15 à 25 % du fer héminique est absorbé contre 2 à 20 % pour le fer non héminique.

La carence en fer peut avoir plusieurs origines : augmentation des besoins chez un individu, diminution des apports, malabsorption, saignements chroniques et pathologies diverses.

Les carences en fer sont généralement traitées par des préparations orales à base de fer (comprimés, gélules, poudre, gouttes, sirop etc.). Lors de l'ingestion par la voie orale, la préparation atteint l'estomac, puis, le fer est absorbé par la muqueuse intestinale et entre ainsi dans la circulation sanguine.

Le traitement de supplémentation en fer par la voie orale peut cependant présenter plusieurs inconvénients comme par exemple celui d'être mal toléré au niveau digestif. Les personnes traitées peuvent se plaindre de maux de ventre car certaines préparations libèrent le fer dès l'estomac. L'intestin ne pouvant absorber qu'une quantité limitée de fer (au maximum 20 à 25% pour le fer héminique), une partie relativement importante du fer ingéré est à nouveau excrétée. Ainsi, le fer administré par la voie orale expose à des effets indésirables fréquents, à savoir des nausées, des diarrhées ou une constipation et des selles noires, plus rarement des douleurs abdominales, un goût métallique dans la bouche ou une coloration noirâtre des dents qui disparait à l'arrêt du traitement.

La prise orale de fer nécessite également de tenir compte de possibles interactions médicamenteuses et alimentaires. En effet, certains médicaments et aliments sont susceptibles de fixer le fer oral dans le tube digestif en formant des complexes non absorbables, ce qui diminue fortement son absorption.

Par ailleurs, il est également fréquent que les préparations orales en fer ne puissent pas être utilisées chez certains types de patients, par exemple chez des patients qui présentent des troubles de l'absorption du fer en raison notamment d'une maladie inflammatoire chronique de l'intestin. Il arrive également que les préparations orales en fer ne soient tout simplement pas efficaces dans le sens où la supplémentation en fer n'améliore pas la carence en fer du sujet, notamment parce que le fer ne passe pas la barrière intestinale.

Ainsi, lorsqu'il n'est pas/plus possible d'administrer les préparations orales de fer, pour les raisons notamment susmentionnées ou parce qu'il existe un besoin clinique d'administrer du fer rapidement ou parce que le dosage recommandé de fer chez un sujet est supérieur à la capacité d'absorption de fer par le tube digestif, alors il est possible d'administrer le fer par voie intraveineuse, ce qui signifie que le fer est administré directement dans le sang par une perfusion intraveineuse.

L'avantage de la voie intraveineuse est que le fer rejoint directement la circulation sanguine, et de là, l'ensemble de l'organisme. Cependant l'administration par voie intraveineuse comporte des effets secondaires potentiels tels que réactions d'hypersensibilité, ostéomalacie hypophosphatémique, atteinte de la fonction hépatique ou rénale, infection, extravasation lors de la perfusion etc., qui rendent indispensable l'administration du fer en hôpital. Cela nécessite donc une hospitalisation avec le besoin structurel inhérent à ce type de soin (lit disponible, personnel médical et infirmier). De plus les risques iatrogènes de ce type de perfusion ne sont pas nuls.

Au vu de ces difficultés, il subsiste à ce jour la nécessité de trouver une alternative aux traitements intraveineux ou oraux du fer.

Dans la présente invention il est proposé de nouveaux nanoclusters de fer pour lutter contre tous types de carences en fer, lesdits nanoclusters étant avantageusement administrés par la voie orale, sans présenter les inconvénients susmentionnés.

Il a déjà été proposé dans la littérature des nanoclusters de fer pour lutter contre les carences en fer. Ainsi, le document US 2016/0022733 décrit des nanocomposites d'oxyde de fer coiffés par de l'acide folique, de l'acide nicotinique et de l'acide ascorbique pour leur utilisation dans le traitement de l'anémie, lesdits nanocomposites étant destinés à une administration orale. Le document US 2016/008292 décrit des nanoparticules d'oxyde de fer recouvertes par des polymères biocompatibles possédant des groupes polyéthylène glycol et silane, lesdits groupes étant liés de façon covalente par l'intermédiaire d'un groupe liant. Ces nanoparticules sont proposées pour le traitement de l'anémie par voie parentérale.

Il est cependant du mérite des Inventeurs d'avoir mis au point de nouveaux nanoclusters de fer présentant des caractéristiques particulièrement appropriées pour lutter contre les carences en fer. Il est également du mérite des Inventeurs d'avoir mis au point un procédé de synthèse original des nanoclusters de fer.

### Résumé

La présente invention concerne des nanoclusters de fer qui présentent les caractéristiques suivantes :
- ils sont recouverts à leur surface d'une couche mixte comprenant de l'histidine (His), des ions acétate (Ac) et des ions ascorbate (Asc),
- ils présentent une forme sphérique,
- ils présentent un diamètre hydrodynamique allant de 0,6 à 2,0 nm, et de préférence inférieur à 1,0 nm,
- ils présentent un diamètre de coeur métallique allant de 0,5 à 1,5 nm, et de préférence inférieur à 1,0 nm,
- ils présentent une stabilité dans le temps allant de 5 à 20 semaines lorsque les nanoclusters sont sous forme liquide et sont stockés à une température de 4°C,
- ils présentent une stabilité dans le temps d'au moins 12 mois, et de préférence de 12 à 18 mois lorsque les nanoclusters sont sous forme sèche et sont stockés à une température de 4°C et sous azote,
- ils présentent des propriétés spectrophotométriques, avec un épaulement sur le spectre UV-Visible à 300 ± 15 nm et un spectre de fluorescence avec des longueurs d'onde d'excitation de 364 ± 15 nm et des longueurs d'onde d'émission de 415 ± 15 nm,
lesdits nanoclusters de fer étant pouvant être désignés par la formule « FeNC@HisAcAsc ». Les nanoclusters de l'invention pourront cependant indifféremment être désignés dans ce qui suit par « nanoclusters », « nanoclusters de fer », « nanoclusters FeNC », « FeNC », « NC-Fe », (« FeNC » ou « NC-Fe » signifiant « nanoclusters de fer »), « nanoclusters FeNC@HisAcAsc », « FeNC@HisAcAsc ».

La formule « FeNC@HisAcAsc » est la plus explicite puisqu'elle décrit que le nanocluster de fer comprend à sa surface une couche comprenant à la fois de l'histidine, des ions acétate et ascorbate.

L'invention concerne également un procédé de préparation desdits nanoclusters de fer qui comprend les étapes suivantes :
- réaction entre de l'acétate de fer (II) et de l'histidine afin d'obtenir un mélange d'acétate de fer et d'histidine, le ratio molaire histidine/acétate de fer (II) étant supérieur ou égal à 8, de préférence va de 8 à 200, et plus préférentiellement encore va de 80 à 200,
- réaction entre le mélange d'acétate de fer et d'histidine avec de l'acide ascorbique afin d'obtenir un mélange d'acétate de fer, d'histidine et d'acide ascorbique, le ratio molaire acide ascorbique/acétate de fer (II) étant supérieur ou égal à 12, de préférence va de 12 à 700, et plus préférentiellement encore va de 130 à 700,
- récupération des nanoclusters de fer recouverts à leur surface d'une couche mixte comprenant de l'histidine, des ions acétate et des ions ascorbate.

L'invention concerne encore les nanoclusters de fer pour une utilisation :
- dans la prévention et/ou le traitement de pathologies générant un déficit en fer (comme par exemple l'anémie ferriprive),
- pour lutter contre les carences en fer.

Enfin, l'invention concerne également une composition comprenant les nanoclusters de fer de l'invention, ladite composition étant un médicament, un complément alimentaire ou une composition alimentaire.

La composition de l'invention est encore caractérisée en ce qu'elle se trouve sous une forme adaptée pour une administration par voie orale.

### Brève description des dessins

D'autres caractéristiques, détails et avantages apparaîtront à la lecture de la description détaillée ci-après et à l'analyse des figures annexées.
La **figure 1** est une représentation schématique d'un nanocluster de fer « FeNC@HisAcAsc » de l'invention, constitué d'un cœur métallique de fer entouré d'une couronne mixte comprenant de l'histidine, des ions acétate et ascorbate.
La **figure 2** est une analyse par chromatographie liquide haute performance (partage en phases inversées) des nanoclusters de fer, montrant la présence d'ions d'acétate à la surface du cœur métallique de fer.
   Le chromatogramme a été obtenu sur des fractions préalablement purifiées (chromatographie d'exclusion stérique) de nanoclusters de fer. Un chromatogramme d'une solution de référence d'acétate de sodium a également été effectué.
La **figure 3** est une analyse par chromatographie liquide haute performance (partage en phases inversées) des nanoclusters de fer, montrant la présence d'histidine et d'ions ascorbate à la surface du cœur métallique de fer.
   Le chromatogramme a été obtenu sur des fractions préalablement purifiées (chromatographie d'exclusion stérique) de nanoclusters de fer. Un chromatogramme d'une solution de référence d'histidine et d'acide ascorbique a également été effectué.
La **figure 4** illustre le diamètre hydrodynamique (en nanomètre) des nanoclusters de fer évalué par diffusion dynamique de la lumière.
La **figure 5** illustre le diamètre hydrodynamique (en nanomètre) des nanoclusters de fer évalué par dispersion de Taylor.
La **figure 6** un spectre UV-Visible des nanoclusters de fer.
La **figure 7** est un spectre de fluorescence des nanoclusters de fer.
La **figure 8** illustre les résultats du test de viabilité (MTT) sur les cellules HepG2 (cellules d'hépatocarcinome) qui ont proliféré en présence de fer, ledit fer se trouvant sous la forme de nitrate de fer (III) (témoin, standard) (représenté par FeNO₃) ou sous la forme des nanoclusters de fer de l'invention à des concentrations variables (représentés par NC-Fe 1x ; NC-Fe 1/2, NC-Fe 1/4, NC-Fe 1/8, NC-Fe 1/16 et NC-Fe 1/32).

Le « Négatif » correspond au milieu de culture sélectif « IMDM » non supplémenté en fer. L'histogramme avec des hachures horizontales tout à gauche de chaque groupe d'histogrammes correspond au jour J1+3 du test de viabilité cellulaire. L'histogramme juste à côté correspond au jour J1+5, et celui d'après correspond au jour J1+7. L'histogramme noir tout à droite de chaque groupe d'histogrammes correspond au jour J1+10.

### Description détaillée

### Les nanoclusters de fer

Ainsi la présente invention concerne des nanoclusters de fer caractérisés en ce qu'ils:
- sont recouverts à leur surface d'une couche mixte comprenant de l'histidine (His), des ions acétate (Ac) et des ions ascorbate (Asc),
- présentent une forme sphérique,
- présentent un diamètre hydrodynamique allant de 0,6 à 2,0 nm, et de préférence inférieur à 1,0 nm,
- présentent un diamètre de cœur métallique allant de 0,5 à 1,5 nm, et de préférence inférieur à 1,0 nm,
- présentent une stabilité dans le temps allant de 5 à 20 semaines lorsque les nanoclusters sont sous forme liquide et sont stockés à une température de 4°C,
- présentent une stabilité dans le temps d'au moins 12 mois, et de préférence de 12 à 18 mois lorsque les nanoclusters sont sous forme sèche et sont stockés à une température de 4°C et sous azote,
- présentent des propriétés spectrophotométriques, avec un épaulement sur le spectre UV-Visible à 300 ± 15 nm et un spectre de fluorescence avec des longueurs d'onde d'excitation de 364 ± 15 nm et des longueurs d'onde d'émission de 415 ± 15 nm,
lesdits nanoclusters de fer pouvant être désignés par la formule « FeNC@HisAcAsc ».

Les nanoclusters de fer objets de l'invention sont des nanoclusters métalliques. Un nanocluster métallique consiste en l'association de dizaines d'atome d'élément métallique (en l'occurrence le fer dans l'invention) avec un diamètre de cœur métallique inférieur ou égal à 2,0 nanomètres (nm).

Les nanoclusters de l'invention sont constitués d'un cœur métallique de fer couvert/recouvert/entouré d'une couche/couronne mixte comprenant de l'histidine, des ions acétate et des ions ascorbate.

On peut employer indifféremment dans la demande les termes « couronne » ou « couche ». Le terme « mixte » est employé pour indiquer que la couronne ou la couche qui entoure le cœur de fer comprend à la fois de l'histidine, des ions acétate et ascorbate.

De même, on peut employer indifféremment les verbes « couvrir/recouvrir/entourer » pour indiquer que le cœur de fer comprend sur l'ensemble de sa surface une couche/couronne d'histidine, d'ions acétate et d'ions ascorbate.

L'ensemble du nanocluster de fer présente une forme sphérique.

La formule « FeNC@HisAcAsc » au sens de l'invention désigne un nanocluster constitué d'un cœur métallique de fer recouvert de ladite couche mixte d'histidine, d'ions acétate et ascorbate. Les nanoclusters de fer de l'invention comprennent ainsi avantageusement trois ligands à la surface du cœur fer, à savoir l'histidine, les ions acétate et ascorbate. Ces trois ligands sont liés au cœur métallique de fer par des liaisons de coordination.

La couronne mixte comprenant l'histidine, les ions acétate et ascorbate confère notamment aux nanoclusters de l'invention une très grande stabilité et une faible réactivité.

Une « faible réactivité » signifie une faible dégradation en particulier liée à l'oxydation (par exemple due au dioxygène de l'air).

La stabilité des nanoclusters de l'invention signifie un maintien de la structure et des propriétés des nanoclusters dans le temps à une température de conservation de 4°C. Le maintien de la structure signifie notamment que la composition du nanocluster (cœur métallique entouré de la couche/couronne mixte telle que ci-dessus définie), sa forme et son diamètre (de cœur métallique et hydrodynamique) sont conservés au cours du temps.

La « forme liquide » des nanoclusters de fer désigne une solution ou un mélange liquide de nanoclusters de fer. La stabilité de 5 à 20 semaines mentionnée ci-dessus concerne les nanoclusters de fer sous forme liquide lorsqu'ils sont stockés à une température de conservation de 4°C.

On entend par « forme sèche » une forme solide qui peut être réduite en poudre si nécessaire. La stabilité de 12 à 18 mois mentionnée ci-dessus concerne les nanoclusters de fer sous forme sèche lorsqu'ils sont stockés à une température de conservation de 4°C et sous azote.

En fonction de leur forme (liquide ou solide), leur stabilité dans le temps sera donc différente. Les nanoclusters de l'invention présentent des propriétés spectrophotométriques, en particulier de fluorescence, qui sont caractéristiques de cette échelle, à savoir un diamètre de cœur métallique inférieur ou égal à 2 nm, qui est intermédiaire entre la molécule et la nanoparticule.

Le diamètre de cœur métallique ou diamètre métallique désigne, comme son nom l'indique, le diamètre formé uniquement par le métal fer.

Le diamètre hydrodynamique prend en compte le diamètre du cœur fer additionné de sa couche/couronne d'histidine, d'ions acétate et ascorbate. Le diamètre hydrodynamique désigne donc le diamètre de la globalité du nanocluster de fer.

Selon un mode de réalisation de l'invention, les nanoclusters de fer présentent un diamètre de cœur métallique et un diamètre hydrodynamique quasi équivalents, de préférence inférieur à 1,0 nm.

Cependant le diamètre de cœur métallique sera bien entendu toujours inférieur au diamètre hydrodynamique.

Le diamètre de cœur métallique est évalué par microscopie électronique à transmission alors que le diamètre hydrodynamique est évalué par diffusion dynamique de la lumière et/ou par dispersion de Taylor.

Selon un mode de réalisation avantageux de l'invention, les nanoclusters de fer se présentent sous forme liquide ou sous forme sèche.

La forme sèche des nanoclusters est avantageuse notamment en ce qu'elle permet un stockage, une conservation et un transport facile.

Selon encore un autre mode de réalisation avantageux, les nanoclusters de fer de l'invention sont caractérisés en qu'ils présentent au moins une des caractéristiques suivantes:
- ils sont aptes à passer la barrière intestinale,
- ils présentent une bonne biodisponibilité,
- ils sont biocompatibles,
- ils sont biodégradables,
- ils sont lyophilisables,
- ils ne sont pas toxiques pour l'organisme humain,
- ils ne s'accumulent pas dans les organes tels que le foie, la rate, les reins ou les poumons. Selon un mode de réalisation avantageux, les nanoclusters de fer de l'invention présentent l'ensemble des caractéristiques ci-dessus décrites.

Le fait que les nanoclusters de l'invention ne soient pas séquestrés dans les dits organes est notamment dû à leur faible taille (diamètre hydrodynamique inférieur ou égal à 2,0 nm, et de préférence inférieur à 1,0 nm). La taille des nanoclusters de l'invention permet une circulation dans le sang plus longue, comparée à des composés de taille plus importante. Plus particulièrement, la petite taille des nanoclusters leur permet de traverser les membranes (en particulier digestive) sans passer par les systèmes d'absorption physiologique via un phénomène de persorption (passage spontané via les pores d'un système physiologique). Ce phénomène de persorption est à l'origine des risques de toxicité des nanoclusters, mais il devient une modalité thérapeutique si on contrôle l'aspect quantitatif des apports de nanoclusters.

Les nanoclusters de l'invention possèdent des propriétés de surface les rendant capable de traverser la barrière intestinale, ce qui représente un avantage important par rapport à des préparations orales de fer souvent inaptes à passer la barrière intestinale.

La « bonne biodisponibilité » signifie que les nanoclusters de fer administrés par voie orale atteignent bien la circulation générale et sont bien distribués aux organes cibles.

On entend par « biocompatibilité » le fait que les nanoclusters de fer sont bien acceptés par les différents organes de l'organisme sans être toxiques pour ces organes.

Le fait que les nanoclusters soient biodégradables signifie que leur dégradation libère des substances qui sont métabolisées ou éliminées sans problème par l'organisme (fer, histidine, acétate et ascorbate).

Selon un mode de réalisation avantageux de l'invention les nanoclusters sont lyophilisables. Il est en effet possible de les lyophiliser car ils sont parfaitement stables. La lyophilisation permet ainsi de stocker, conserver et transporter facilement les nanoclusters. La stabilité des nanoclusters est telle que définie ci-dessus.

Les propriétés avantageuses des nanoclusters de l'invention sont notamment dues à la combinaison originale de ses constituants, à savoir le fer, l'histidine, les ions acétate et ascorbate.

A la connaissance des Inventeurs il n'a en effet jamais été décrit à ce jour des nanoclusters de fer comprenant une couronne/couche mixte d'histidine, d'ions acétate et ascorbate entourant un noyau métallique de fer, et qui présentent les propriétés avantageuses ci-dessus décrites.

### Le procédé de préparation des nanoclusters de fer

La présente invention a encore pour objet un procédé de préparation de nanoclusters de fer tels que ci-dessus définis, caractérisé en ce qu'il comprend les étapes suivantes :
- réaction entre de l'acétate de fer (II) et de l'histidine afin d'obtenir un mélange d'acétate de fer et d'histidine, le ratio molaire histidine/acétate de fer (II) étant supérieur ou égal à 8, de préférence va de 8 à 200, et plus préférentiellement encore va de 80 à 200.
- réaction entre le mélange d'acétate de fer et d'histidine avec de l'acide ascorbique afin d'obtenir un mélange d'acétate de fer, d'histidine et d'acide ascorbique, le ratio molaire acide ascorbique/acétate de fer (II) étant supérieur ou égal à 12, de préférence va de 12 à 700, et plus préférentiellement encore va de 130 à 200,
- récupération des nanoclusters de fer.

Les ratios molaires tels que définis ci-dessus, respectivement entre l'histidine et l'acétate de fer, et entre l'acide ascorbique et l'acétate de fer, sont importants en ce sens qu'ils permettent aux ligands histidine, ions acétate et ascorbate de se lier au cœur métallique de fer. On obtient de cette manière des nanoclusters comprenant trois ligands à la surface du cœur fer, ces trois ligands étant liés à la surface du cœur de fer par des liaisons de coordination.

L'acide ascorbique est un réducteur. La réaction entre le mélange d'acétate de fer et d'histidine avec l'acide ascorbique est plus particulièrement une réaction de réduction du mélange d'acétate de fer et d'histidine avec de l'acide ascorbique. L'acide ascorbique permet notamment d'obtenir des nanoclusters de fer dépourvus de toute toxicité.

La présente invention résulte notamment de la découverte inattendue des Inventeurs que la combinaison originale des réactifs utilisés, à savoir l'acétate de fer, l'histidine et l'acide ascorbique, et dans les proportions telles que ci-dessus définies, permet d'obtenir des nanoclusters de fer aux propriétés particulièrement avantageuses.

L'excellente stabilité des nanoclusters de l'invention en est un exemple.

Selon un mode de réalisation de l'invention, les nanoclusters de fer peuvent être plus particulièrement préparés selon le protocole « en solution » ou selon le protocole « en phase solide ». Chacune de ces deux voies de synthèse est conforme au procédé ci-dessus décrit.

### 1/ Protocole en solution

Selon un mode de réalisation avantageux de l'invention, le procédé de préparation tel que ci-dessus défini est plus particulièrement caractérisé en ce qu'il est effectué sous gaz inerte et en ce que :
- l'acétate de fer est sous forme de solution et l'histidine est sous forme de poudre,
- une solution d'acétate de fer et d'histidine est préparée par ajout d'histidine dans la solution d'acétate de fer,
- la solution d'acétate de fer et d'histidine est ajustée à une valeur de pH allant de 11 à 13, et de préférence est de 12,
- l'acide ascorbique est sous forme de poudre,
- une solution d'acétate de fer, d'histidine et d'acide ascorbique est préparée par ajout d'acide ascorbique à la solution d'acétate de fer et d'histidine dont le pH a été ajusté aux valeurs susmentionnées,
- la solution d'acétate de fer, d'histidine et d'acide ascorbique est agitée pendant 2h à 6h, et de préférence 4h, à une température allant de 35°C à 45°C, et de préférence 40°C,
- une solution comprenant des nanoclusters de fer est obtenue à l'issue de l'étape précédente d'agitation,
- la solution qui comprend les nanoclusters de fer est éventuellement dialysée afin d'obtenir une solution purifiée de nanoclusters de fer,
- la solution qui comprend les nanoclusters de fer, éventuellement dialysée, est éventuellement lyophilisée afin d'obtenir une forme sèche de nanoclusters de fer.

La solution comprenant les nanoclusters de fer, éventuellement dialysée, présente une stabilité dans le temps allant de 5 à 20 semaines à une température de conservation de 4°C. La dialyse permet notamment de supprimer tout ce qui n'est pas lié au coeur métallique de fer, comme par exemple éventuellement un excès d'histidine ou d'acide ascorbique ou encore du fer résiduel éventuellement présent dans la solution de nanoclusters. La couche comprenant l'histidine, les ions acétate et ascorbate est liée au coeur métallique de fer par des liaisons de coordination.

La forme sèche des nanoclusters de fer, obtenue à l'issue de la lyophilisation, présente une stabilité dans le temps d'au moins 12 mois, et de préférence de 12 à 18 mois, à une température de conservation de 4°C et sous azote.

La forme sèche des nanoclusters de fer peut être reconstituée à tout moment, par mélange dans un solvant de reconstitution, comme par exemple de l'eau purifiée. On entend par « reconstituer/reconstitution » l'opération de mélange simple de la forme sèche ou du lyophilisat avec un solvant.

L'analyse de la solution de nanoclusters de fer, obtenue à l'issue de la reconstitution de la forme sèche, montre que les nanoclusters de fer présentent l'ensemble des propriétés définies ci-dessus et sont donc exactement les mêmes que ceux directement obtenus à l'issue de leur procédé de préparation.

La solution de nanoclusters de fer, obtenue à l'issue de la reconstitution de la forme sèche, présente une stabilité dans le temps allant de 5 à 12 semaines à une température de conservation de 4°C, et de préférence sous azote.

Le procédé de préparation tel que ci-dessus défini est encore caractérisé en qu'il comprend en outre au moins une caractéristique choisie parmi :
- le gaz inerte est de l'azote,
- la solution d'acétate de fer est préparée par ajout d'acétate de fer dans de l'eau ultra pure filtrée,
- la solution d'acétate de fer présente une concentration allant de 0,5 à 5,0 mM,
- la concentration d'histidine est supérieure à la concentration de la solution d'acétate de fer,
- le pH de la solution d'acétate de fer et d'histidine est ajusté à l'aide d'hydroxyde de sodium,
- la concentration d'acide ascorbique est égale à la concentration d'histidine,
- la solution qui comprend les nanoclusters de fer, éventuellement dialysée, présente une concentration en fer allant de 14 à 112 µg/mL,
- la solution qui comprend les nanoclusters de fer, éventuellement dialysée, est lyophilisée afin d'obtenir une forme sèche de nanoclusters de fer.

Selon un mode de réalisation avantageux, le procédé de l'invention présente l'ensemble des caractéristiques ci-dessus décrites.

### 2/ Protocole en phase solide

Selon un autre mode de réalisation avantageux de l'invention, le procédé de préparation des nanoclusters de fer tel que ci-dessus défini est plus particulièrement caractérisé en ce que :
- l'acétate de fer est sous forme de poudre et l'histidine est sous forme de poudre,
- un mélange pulvérulent d'acétate de fer et d'histidine est obtenu par mélange de chacune des poudres d'acétate de fer et d'histidine,
- le mélange pulvérulent d'acétate de fer et d'histidine est broyé jusqu'à obtention d'un mélange pulvérulent homogène en couleur,
- le mélange pulvérulent homogène d'acétate de fer et d'histidine est placé dans un réacteur,
- l'acide ascorbique est sous forme de poudre,
- l'acide ascorbique est ajouté dans le réacteur comprenant le mélange pulvérulent homogène d'acétate de fer et d'histidine,
- le mélange pulvérulent d'acétate de fer, d'histidine et d'acide ascorbique ainsi obtenu est mis sous agitation, puis de l'eau est ajoutée goutte à goutte dans le réacteur, ladite eau étant de l'eau ultra pure filtrée,
- le réacteur est mis sous gaz inerte et à l'abri de la lumière,
- le mélange d'acétate de fer, d'histidine, d'acide ascorbique et d'eau est maintenu sous agitation dans le réacteur pendant 16 à 36h, et de préférence 24h,
- un mélange liquide qui comprend les nanoclusters de fer est obtenu à l'issue de l'étape précédente d'agitation,
- le mélange liquide qui comprend les nanoclusters de fer est éventuellement dialysé afin d'obtenir un mélange liquide purifié de nanoclusters de fer,
- le mélange liquide qui comprend les nanoclusters de fer, éventuellement dialysé, est éventuellement lyophilisé afin d'obtenir une forme sèche de nanoclusters de fer.

Le procédé de préparation tel que ci-dessus défini est encore caractérisé en qu'il comprend en outre au moins une caractéristique choisie parmi :
- la concentration d'histidine est supérieure à la concentration d'acétate de fer,
- la concentration d'acide ascorbique est égale à la concentration d'histidine,
- l'eau ajoutée dans le réacteur est de l'eau ultra pure filtrée,
- le gaz inerte est de l'azote,
- le mélange liquide qui comprend les nanoclusters de fer, éventuellement dialysé, présente une concentration en fer allant de 1500 à 15000 µg/mL,
- le mélange liquide qui comprend les nanoclusters de fer, éventuellement dialysé, est lyophilisé afin d'obtenir une forme sèche de nanoclusters de fer.

Selon un autre mode de réalisation de l'invention, la forme sèche de nanoclusters de fer obtenue à l'issue de la lyophilisation (selon le protocole « solution » ou le protocole « en phase solide ») est conservée sous azote, de préférence dans des flacons, et de préférence à 4°C. Dans de telles conditions, la poudre de nanoclusters de fer pourra être conservée pendant une durée d'au moins 12 mois, et de préférence de 12 à 18 mois, sans altération de la stabilité des nanoclusters de fer.

La reconstitution de la poudre de nanoclusters à l'issue de cette période montre que les nanoclusters sont les mêmes que ceux directement obtenus à l'issue de leur préparation (selon le protocole « solution » ou le protocole « en phase solide »). En effet les nanoclusters de fer présentent l'ensemble des propriétés définies ci-dessus.

### L'utilisation des nanoclusters de fer

L'invention a encore pour objet les nanoclusters de fer tels que ci-dessus définis ou obtenus selon les procédés tels que ci-dessus définis, pour une utilisation comme médicament.

Plus particulièrement, l'invention a pour objet les nanoclusters de fer tels que ci-dessus définis ou obtenus selon les procédés tels que ci-dessus définis, pour une utilisation dans la prévention et/ou le traitement de pathologies générant un déficit en fer.

A titre d'exemple de pathologie générant un déficit en fer est une anémie ferriprive. L'invention concerne encore les nanoclusters de fer tels que ci-dessus définis ou obtenus selon les procédés tels que ci-dessus définis, pour une utilisation pour lutter contre les carences en fer.

Dans la présente demande, une carence en fer désigne une carence en fer au sens large, à savoir une carence en fer accompagnée ou non d'une anémie ferriprive.

Un autre objet de l'invention réside en une composition caractérisée en ce qu'elle comprend des nanoclusters de fer tels que ci-dessus définis ou obtenus selon les procédés tels que ci-dessus définis.

La composition de l'invention pourra être un médicament, un complément alimentaire ou une composition alimentaire.

La quantité de fer par composition peut déterminer s'il s'agit plutôt d'un complément alimentaire ou plutôt d'un médicament. Ainsi, un complément alimentaire devrait comprendre moins de fer qu'un médicament.

A titre d'exemple de composition alimentaire on peut par exemple citer des laits infantiles qui sont supplémentés en fer, et plus particulièrement avec les nanoclusters de fer de l'invention.

Selon un mode de réalisation avantageux de l'invention, la composition se trouve sous une forme adaptée pour une administration par voie orale.

Les nanoclusters de fer peuvent avantageusement être administrés par la voie orale car ils sont aptes à passer la barrière intestinale sans difficulté, notamment du fait de leur petite taille.

L'utilisation des nanoclusters de fer de l'invention permet avantageusement de s'affranchir de la voie intraveineuse.

Selon encore un mode de réalisation avantageux, la composition de l'invention comprenant les nanoclusters de fer comprend une quantité de fer inférieure à la quantité de fer habituellement présente dans une préparation orale classique, qu'il s'agisse d'un médicament ou d'un complément alimentaire.

Avantageusement, la composition de l'invention ne présente pas les inconvénients pouvant être rencontrés avec une préparation orale classique, qu'il s'agisse d'un médicament ou d'un complément alimentaire.

### Exemples

Les exemples ci-après illustrent l'invention, ils ne la limitent en aucune façon.

### Exemple 1

### Préparation des nanoclusters de fer

Cet exemple décrit respectivement les deux voies de synthèse possibles pour préparer les nanoclusters de fer de l'invention, à savoir le « protocole en solution » et le « protocole en phase solide ».

### 1/ Protocole en solution

### Réactifs utilisés :

- Acétate de fer (II) [Fe(CH₃COO)₂], M = 171,83 g/mol (Sigma Aldrich, Cas 3094-87-9) ;
- L(-)-Histidine, M=155,15 g/mol (Merck, Cas 71-00-1) ;
- Acide ascorbique, M= 176,12 g/mol (Sigma Aldrich, Cas 50-81-7);
- Solution de NaOH 1M, M= 40,00 g/mol (VWR, Cas 1310-73-2).

### Précautions à prendre

La synthèse est réalisée sous gaz inerte (azote). La verrerie est lavée à l'eau régale (1 volume d'acide nitrique 65% pour 2 volumes d'acide chlorhydrique 37%).

L'eau ultra pure est utilisée et est filtrée sur un filtre de diamètre de pores 0,2 µm.

L'acétate de fer se trouve sous forme de poudre et est conservé sous azote. Une fois le fer pesé, le stock restant doit être remis sous azote.

Les nanoclusters de fer étant destinés à être étudiés in vivo, il est nécessaire de travailler sous une hotte propre et de nettoyer tous les équipements utilisés avec de l'éthanol 70% v/v.

### Préparation d'une solution mère d'acétate de fer 2,5 mM

Une quantité de 42,9 mg d'acétate de fer est disposée dans une fiole jaugée de 100 mL. De l'eau ultra pure filtrée est ajoutée jusqu'au trait de jauge de la fiole. Une solution d'acétate de fer d'une concentration de 2,5 mM est obtenue.

Après complète solubilisation, la solution d'acétate de fer est transvasée dans un flaconnage adapté. Cette solution peut être conservée pendant un mois dans le réfrigérateur à 4°C.

### Synthèse des nanoclusters de fer stabilisés avec de l'histidine

Une quantité de 500 µL de la solution mère d'acétate de fer telle que préparée à l'étape précédente est ajoutée dans un ballon à col rond pouvant contenir jusqu'à 50 mL de solution. Puis, une quantité de 4500 µL d'eau ultra pure filtrée est ajoutée dans le ballon. La solution d'acétate de fer obtenue est appelée 1x.

La solution d'acétate de fer 1x est agitée à 130 rpm avec l'agitateur multi plaques. Une quantité de 39 mg d'histidine est ajoutée à la solution d'acétate de fer. La solution d'acétate de fer et d'histidine est agitée pendant 15 minutes.

La solution prend une teinte légèrement rouge. Après 15 minutes d'agitation le pH de la solution d'acétate de fer et d'histidine est ajusté à 12 avec 10 gouttes de NaOH 1M. Une quantité de 139 mg d'acide ascorbique est ajoutée au mélange réactionnel. On attend 2 minutes la complète solubilisation de l'acide ascorbique. Le ballon (réacteur) est placé dans un bain marie à 40°C sous agitation (vitesse réglée à 6) pendant 4 heures.

A l'issue de la synthèse, la solution de nanoclusters obtenue est incolore. La solution de nanoclusters de fer ainsi obtenue est appelée **1x** et présente une concentration en fer de 14 µg/mL. Elle est conservée au frais, à 4°C.

Le rendement de synthèse est de 100% : il n'y a pas de fer résiduel (élément fer) dans la solution de nanoclusters. Les solutions de nanoclusters de fer peuvent être lyophilisées. Des solutions d'acétate de fer d'une concentration allant de **1x** à **8x** sont préparées afin d'obtenir des solutions de nanoclusters de fer **1x** à **8x** qui présentent une concentration en fer allant de 14 à 112 µg/mL.

A titre indicatif une solution d'acétate de fer **2x** est préparée en disposant une quantité de 1000 µL de solution mère d'acétate de fer dans le ballon et en complétant à 5000 µL avec de l'eau ultra pure filtrée. Une solution d'acétate de fer **4x** est préparée en disposant une quantité de 2000 µL de solution mère d'acétate de fer dans le ballon et en complétant à 5000 µL avec de l'eau ultra pure filtrée etc.

Les solutions de nanoclusters de fer **1x** à **8x** ainsi obtenues sont conservées au frais à 4°C.

### Dialyse des nanoclusters de fer

La solution de nanoclusters de fer **1x** obtenue à l'étape précédente est purifiée par dialyse.

Une cellule de dialyse est préparée (X12 Float a lyzer G2 CE MWCO 100-500 D, Référence 1511160), et un bécher de 150 mL est rempli avec 100 mL d'eau ultra pure filtrée. La cellule de dialyse est remplie avec de l'eau ultrapure filtrée à l'aide d'une pipette pasteur. La cellule de dialyse est placée dans le bécher sous agitation (130 rpm). On laisse la cellule s'hydrater et se laver pendant 1h.

L'eau est ensuite remplacée par un nouveau volume de 100 mL d'eau ultra pure filtrée. La cellule de dialyse est vidée à l'aide d'une pipette pasteur puis est remplie avec la solution de nanoclusters de fer **1x,** qui est laissée sous agitation toute la nuit (pendant 12h) à une température entre 2 et 6°C.

La solution dialysée de nanoclusters de fer **1x** ainsi obtenue est transférée dans un flaconnage adapté et est conservée à une température de 4°C.

La dialyse n'influe pas sur la concentration en fer des nanoclusters. Ainsi, les concentrations en fer des solutions de nanoclusters de fer dialysées sont identiques à celles des solutions non dialysées.

Les solutions de nanoclusters de fer, éventuellement dialysées, peuvent être lyophilisées. Les concentrations en fer des solutions de nanoclusters de fer dialysées sont identiques à celles des solutions non dialysées, et vont de 14 à 112 µg/mL pour des solutions d'acétate de fer d'une concentration allant de **1x** à **8x.**

### 2/ Protocole en phase solide

### Réactifs utilisés et précautions à prendre

L'acétate de fer (II), la L(-)-Histidine et l'acide ascorbique sont les mêmes que ceux utilisés dans le protocole solution. Dans le protocole en phase solide l'hydroxyde de sodium n'est pas nécessaire.

Les précautions à prendre sont les mêmes que celles pour le protocole en solution.

### Synthèse des nanoclusters de fer stabilisés avec de l'histidine

Une quantité de 23 mg d'acétate de fer est pesée puis est placée à l'intérieur d'un mortier en agate. Une quantité de 1,7 g d'histidine est ensuite pesée. Un volume de poudre d'histidine pour un volume de poudre d'acétate de fer est ajouté en prenant soin de bien broyer les poudres à l'aide du pilon jusqu'à obtenir un mélange de couleur et d'aspect homogène. Cette opération est répétée tant qu'il y a de l'histidine.

Le mélange final des deux poudres doit avoir une couleur rouge et la poudre doit être homogène. Le mélange des deux poudres est ensuite transféré dans un ballon monocol de 50 mL (col rodé NS 19/26) à l'aide d'une spatule. Une quantité de 3 g d'acide ascorbique est pesée et transférée dans le ballon. Un agitateur magnétique en forme d'olive est placé au fond du ballon. Une quantité de 5 mL d'eau ultra pure est filtrée à l'aide d'une seringue en plastique de 5 mL et est ajoutée goutte à goutte dans le réacteur. Un mélange liquide est obtenu.

Le réacteur est fermé à l'aide d'un bouchon à jupe rabattable (diamètre 19,4 mm) et est mis sous azote sans créer de surpression à l'aide d'un ballon de baudruche. Le réacteur est entouré d'un papier aluminium puis est placé sous agitation (200 rpm) le temps de la réaction. Il faut attendre 24h avant la fin de la réaction. A la fin de la réaction, le produit obtenu, qui se trouve sous la forme d'un liquide, a une couleur rouge.

Le liquide obtenu, comprenant les nanoclusters de fer, présente une concentration en fer de 1500 µg/mL, et est appelé **100x.**

La concentration en fer des nanoclusters est bien entendu dépendante de la quantité d'acétate de fer utilisée au départ du procédé de l'invention.

Les opérations ci-dessus décrites sont répétées de sorte à obtenir des concentrations en fer allant de 1500 **(100x)** à 15000 µg/mL **(1000x)** pour des quantités d'acétate de fer au départ du procédé de l'invention (protocole en phase solide) allant de 23 à 230 mg.

Le liquide obtenu comprenant les nanoclusters de fer est ensuite transféré dans un flaconnage en plastique adapté (le volume final n'est pas de 5 mL mais un peu plus, environ 8,5 mL). Le liquide comprenant les nanoclusters de fer est soit stocké à 4°C, soit transféré au lyophilisateur.

La lyophilisation est effectuée par fractions de 1 mL, sans ajout de réactifs supplémentaires. Après la lyophilisation, le contenu du flacon (qui comprend les nanoclusters de fer sous forme sèche) est placé sous azote et est stocké à 4°C.

La forme sèche des nanoclusters de fer peut être reconstituée à tout moment dans 1 mL d'eau purifiée. La solution de nanoclusters de fer ainsi reconstituée est conservée à 4°C, de préférence sous azote.

### Exemple 2

### Caractérisation des nanoclusters de fer

Les nanoclusters de fer tels qu'obtenus à l'exemple 1, qu'il s'agisse du protocole en solution ou du protocole en phase solide, sont caractérisés quant à leur structure, leurs tailles, leurs propriétés spectrophotométriques et leur stabilité.

### Structure des nanoclusters de fer

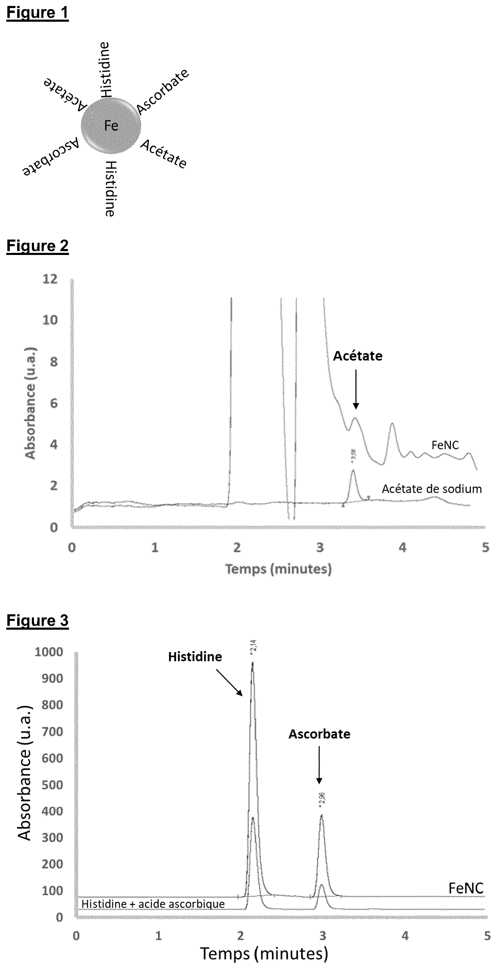

Les nanoclusters de fer de l'invention présentent plus particulièrement une forme sphérique. Ils sont constitués d'un cœur métallique de fer couvert d'une couronne mixte comprenant de l'histidine, des ions acétate et ascorbate. La **figure 1** est une représentation schématique d'un nanocluster de fer de l'invention, qui peut encore être désigné par la formule « FeNC@HisAcAsc ».

La présence d'ions acétate à la surface des nanoclusters de fer a été démontrée par chromatographie liquide haute performance (CLHP), plus particulièrement par chromatographie de partage à polarité de phases inversées.

Les nanoclusters de fer de la solution dialysée **1x** telle qu'obtenue à l'exemple 1, voir point 1/ « Protocole en solution », sont détruits (dissolution totale et retour aux différents éléments composant la structure des nanoclusters) par un procédé chimique, à savoir mise en solution dans un acide concentré (HCl) puis une base concentrée (NaOH), puis sont analysés par CLHP en comparaison à un témoin acétate de sodium.

Les résultats obtenus sont illustrés dans le chromatogramme de la **figure 2****.** Le pic à 3,6 min associé aux ions acétate (témoin, voir tracé du bas) est retrouvé dans les nanoclusters de fer (voir tracé du haut), montrant ainsi la présence d'ions acétate à la surface du cœur métallique.

La présence d'histidine et d'ions ascorbate à la surface des nanoclusters de fer a également été démontrée par chromatographie liquide haute performance, plus particulièrement après purification des nanoclusters en solution par chromatographie d'exclusion stérique, en comparaison à un témoin histidine et acide ascorbique.

Les résultats obtenus sont illustrés dans le chromatogramme de la **figure 3****.**

Le pic à 2,1 min associé à l'histidine (témoin, voir tracé du bas) est retrouvé dans les nanoclusters de fer (voir tracé du haut), montrant ainsi la présence d'histidine à la surface du cœur métallique.

Le pic à 3,0 min associé aux ions ascorbate (témoin, voir tracé du bas) est retrouvé dans les nanoclusters de fer (voir tracé du haut), montrant ainsi la présence d'ions ascorbate à la surface du cœur métallique.

### Taille des nanoclusters de fer

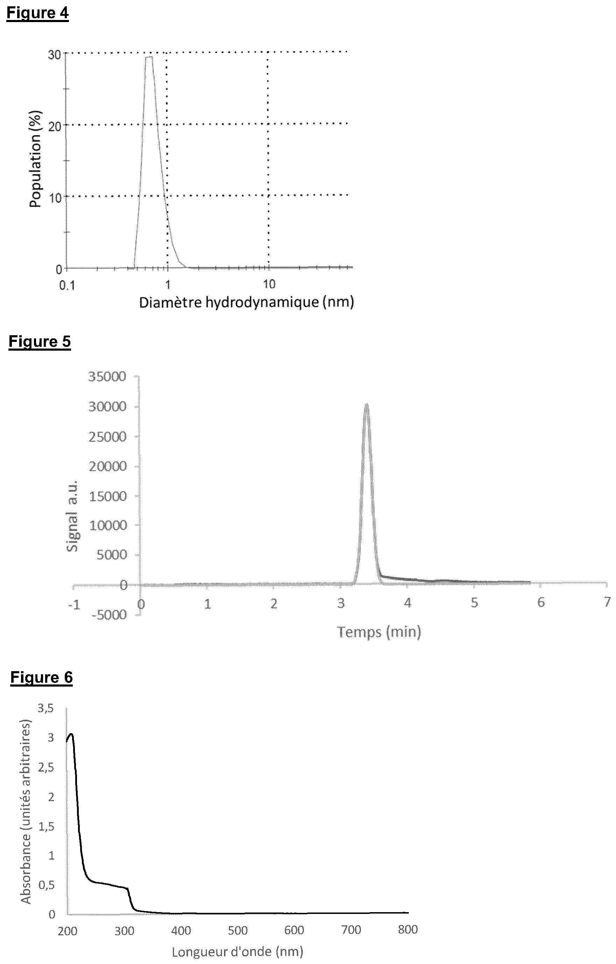

Le diamètre hydrodynamique (Dh) des nanoclusters de fer a été évalué par diffusion dynamique de la lumière **(****figure 4****)** (angle 173°, laser 530 nm, température 25°C sur Nanosizer Malvern) et par analyse par dispersion de Taylor **(****figure 5****).**

La méthode d'analyse par diffusion dynamique de la lumière consiste à analyser le mouvement Brownien des particules et le modéliser à l'aide de l'équation de Stokes-Einstein. La méthode d'analyse par dispersion de Taylor consiste à injecter une bande de soluté dans un tube capillaire ouvert (50 µm) et à la mobiliser sous l'influence d'un flux hydrodynamique (pression positive 1 psi, profil de vitesse parabolique). Le principe de la détermination du rayon hydrodynamique repose sur la relation de Taylor-Aris qui établit le lien entre l'étalement du pic de soluté (modélisation d'une gaussienne) et le coefficient de diffusion moléculaire.

Le diamètre métallique des nanoclusters de fer a été évalué par microscopie électronique à transmission (dépôt sur grilles en nickel, observations sous faisceaux opérant à 200 kV (LaB6 cathode) Philips CM 200).

Les diamètres (hydrodynamique et métallique) des nanoclusters de fer ont respectivement été évalués immédiatement à l'issue de leur synthèse, qu'il s'agisse du protocole en solution ou en phase solide.

Concernant le protocole en solution, les diamètres hydrodynamique et métallique ont été évalués sur la solution de nanoclusters de fer **1x,** non dialysée et non lyophilisée, qui présente une concentration en fer de 14 µg/mL.

Concernant le protocole en phase solide, les diamètres hydrodynamique et métallique ont été évalués sur les échantillons lyophilisés obtenus à partir du liquide de nanoclusters de fer **100x,** non dialysé, qui présente une concentration en fer de 1500 µg/mL.

Le diamètre hydrodynamique moyen des nanoclusters de fer, tout comme le diamètre métallique, est inférieur à 1,0 nm. Plus particulièrement, il ressort des **figures 4** et **5** qu'en diffusion dynamique de la lumière **(****fig. 4****)** et en dispersion de Taylor **(****fig. 5****)** le diamètre hydrodynamique des nanoclusters de fer est égal à 0,69 ± 0,06 nm.

### Propriétés spectrophotométriques

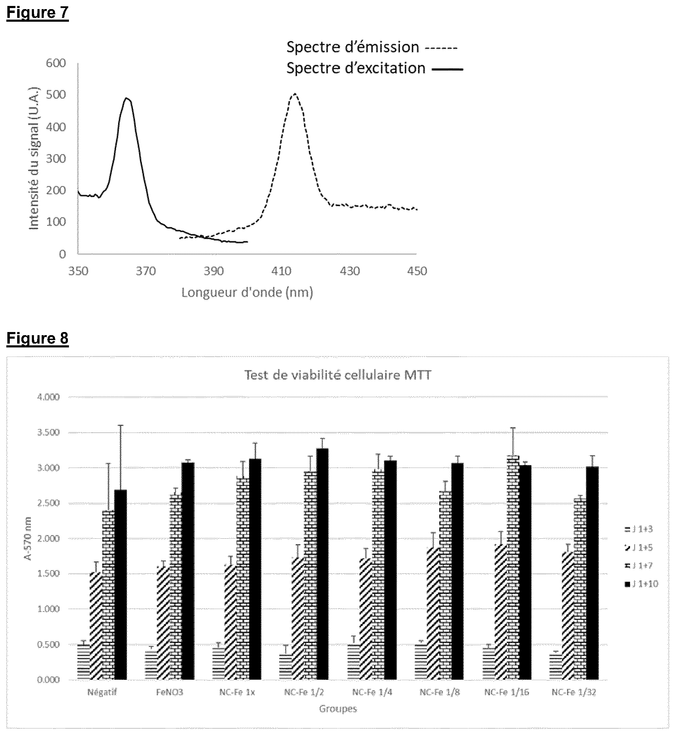

Les propriétés spectrophotométriques des nanoclusters de fer ont été évaluées par spectroscopie UV-Visible **(****figure 6****)** et par spectroscopie de fluorescence **(****figure 7****),** immédiatement à l'issue de leur synthèse.

Le spectre UV-vis montre un épaulement à 300 ± 15 nm **(****fig. 6****),** ce qui confirme l'existence des nanoclusters.

Une fluorescence des nanoclusters de fer existe avec **(****fig. 7****)** une longueur d'onde d'excitation de 364 ± 15 nm et une longueur d'onde d'émission de 415 ± 15 nm, ce qui confirme également l'existence des nanoclusters.

Les nanoclusters de fer de l'invention présentent des propriétés optiques, en particulier de fluorescence, caractéristiques de cette échelle intermédiaire entre la molécule et la nanoparticule.

### Stabilité des nanoclusters de fer

La stabilité des nanoclusters de fer a été évaluée par mesure de leur diamètre hydrodynamique en diffusion dynamique de la lumière (angle 173°, laser 530 nm, température 25°C sur Nanosizer Malvern).

Les analyses ont été effectuées sur les nanoclusters de fer obtenus selon le protocole en solution et selon le protocole en phase solide.

Concernant le protocole en solution, les analyses ont été effectuées sur les solutions de nanoclusters de fer **1x** (concentration en fer de 14 µg/mL). La stabilité a été évaluée un peu plus de 5 semaines après la synthèse desdites solutions.

Il a été constaté que le diamètre hydrodynamique des nanoclusters de fer était toujours de 0,69 ± 0,06 nm plus de 5 semaines après leur synthèse, ce qui démontre leur excellente stabilité.

Concernant le protocole en phase solide, les analyses ont été effectuées sur :
- les échantillons lyophilisés obtenus à partir du liquide de nanoclusters de fer **100x,** non dialysé (concentration en fer de 1500 µg/mL),
- les solutions reconstituées après lyophilisation desdits échantillons, dans le même volume que celui de la lyophilisation.

Le diamètre hydrodynamique des nanoclusters de fer des échantillons lyophilisés est de 0,70 nm après plus de 5 semaines de conservation sous azote.

Après reconstitution des échantillons, dans le même volume que celui de la lyophilisation, le diamètre hydrodynamique des nanoclusters de fer des échantillons reconstitués est de 0,76 nm, ce qui démontre encore une fois leur excellente stabilité.

### Exemple 3

### Evaluation de la toxicité des nanoclusters de fer

Dans cet exemple sont décrits les résultats du test de viabilité (test MTT) sur les cellules HepG2 (cellules d'hépatocarcinome) qui ont proliféré en présence de fer, ledit fer se trouvant sous la forme de nitrate de fer (III) (témoin, standard) ou sous la forme des nanoclusters de fer de l'invention à des concentrations variables.

### Fer

Le nitrate de fer (III) (ou nitrate ferrique) utilisé comme témoin est le composé chimique de formule semi-développée « Fe(NO₃)₃ », qui est plus particulièrement utilisé sous sa forme nonahydrate « Fe(NO₃)₃.9H₂O ».

Une solution de nitrate de fer nonahydrate est préparée pour un volume total de 50 mL à la concentration de 100 mg/L d'eau ce qui correspond à une concentration de 14 µg/mL de fer. Cette solution est filtrée sous hotte de type PSM.

La solution de nanoclusters de fer **1x,** qui comprend une concentration en fer de 14 µg/mL telle que préparée à l'exemple 1 (point 1/ protocole solution), est plus particulièrement utilisée.

Une gamme de dilution en cascade de la solution de nanoclusters de fer **1x** est réalisée au 1/2, 1/4, 1/8, 1/16 et 1/32^{ème} (voir ci-après les milieux 4 à 8 préparés).

### Cellules HepG2

Les cellules HepG2 sont une lignée cellulaire provenant du tissu hépatique d'un patient présentant un carcinome hépatocellulaire (CHC).

### Test MTT

Le test MTT est une méthode colorimétrique rapide permettant de quantifier les cellules vivantes au sein d'un échantillon. Le réactif utilisé est le sel de tétrazolium « MTT » (« bromure de 3-(4,5-diméthylthiazol-2-yl)-2,5-diphényl tétrazolium »). L'anneau de tétrazolium qu'il contient est réduit, par la succinate déshydrogénase mitochondriale des cellules vivantes actives, en formazan. Ceci forme un précipité dans la mitochondrie de couleur violette.

La quantité de précipité formée est proportionnelle à la quantité de cellules vivantes (mais également à l'activité métabolique de chaque cellule). Il suffit donc après l'incubation des cellules avec du MTT pendant un certain temps à 37°C (environ trois heures) de dissoudre les cellules, leurs mitochondries et donc les précipités de Formazan violets dans du DMSO 100 % (diméthylsulfoxyde).

Un simple dosage de la densité optique à 570 nm par spectroscopie permet de connaître la quantité relative de cellules vivantes et actives métaboliquement.

Il est donc nécessaire, dans le cas où le test doit être quantitatif, de réaliser pour chaque essai une courbe d'étalonnage. La lecture est réalisée à 570 nm grâce à un spectrophotomètre (voir **figure 8****).**

### Conditions de croissance des cellules HepG2

Les cellules HepG2 sont mises en croissance dans un milieu de culture complet « DMEM » (milieu de Eagle modifié par Dulbecco) dans des plaques 24 puits pendant 24h, puis le milieu est changé pour un milieu de culture sélectif « IMDM* » (voir ci-après) +/- nanoclusters de fer de l'invention versus le nitrate de fer nonahydrate Fe(NO₃)₃.9H₂O.

Le Fe³⁺ est réduit en Fe²⁺ puis passe la membrane plasmique via « DMT1 » (« divalent metal transporter 1 »).

### Milieu de culture sélectif IMDM*:

- IMDM (milieu de Dulbecco Modifié selon Iscove),
- 10% SVF (sérum de veau fœtal) (dialysé),
- 1% antibiotiques (mélange de pénicilline et de streptomycine),
- 1% (1 mM) pyruvate.

### Huit milieux sélectifs sont préparés :

- Milieu 1 : IMDM*;
- Milieu 2 : IMDM* + 1% Fe(NO₃)₃.9H₂O (stock 100 mg/L);
- Milieu 3: IMDM* + 1% solution nanoclusters de fer **1x** (soit 14 µg/mL équivalent Fer) ;
- Milieu 4 : Dilution au 1/2 Milieu 3 dans IMDM* ;
- Milieu 5 : Dilution au 1/2 Milieu 4 dans IMDM* (soit 1/4 du Milieu 3) ;
- Milieu 6 : Dilution au 1/2 Milieu 5 dans IMDM* (soit 1/8 du Milieu 3) ;
- Milieu 7 : Dilution au 1/2 Milieu 6 dans IMDM* (soit 1/16 du Milieu 3) ;
- Milieu 8 : Dilution au 1/2 Milieu 7 dans IMDM* (soit 1/32 du Milieu 3).

### Durée du test

Une plaque est préparée pour chaque étape (J1+3, J1+5, J1+7, J1+10). La durée globale du test est de 11 jours.

Les étapes sont les suivantes :
- J0 : Ensemencement en p24 (triplicats), 4000 cellules/cm², milieu DMEM complet (500 µL/Puits) ;
- J1 : Changement pour milieu sélectif IMDM solution de nanoclusters de fer versus Fe(NO₃)₃.9H₂O (0,1 mg/L) ;
- J1+3 : 1 plaque arrêtée pour MTT, changement de milieu pour le reste ;
- J1+5 : 1 plaque arrêtée pour MTT, changement de milieu pour le reste ;
- J1+7 : 1 plaque arrêtée pour MTT, changement de milieu pour le reste ;
- J1+10 : 1 plaque arrêtée pour MTT, fin du test.

Les résultats obtenus sont décrits à la **figure 8****,** où :
- « Négatif » correspond au Milieu 1,
- « FeNO3 » correspond au Milieu 2,
- « NC-Fe 1x » correspond au Milieu 3,
- « NC-Fe 1/2 » correspond au Milieu 4,
- « NC-Fe 1/4 » correspond au Milieu 5,
- «NC-Fe 1/8 » correspond au Milieu 6,
- « NC-Fe 1/16 » correspond au Milieu 7,
- « NC-Fe 1/32 » correspond au Milieu 8.

Les tests de viabilité MTT répétés à J+3, J+5, J+7 et J+10 indiquent que le traitement avec les nanoclusters de fer de l'invention n'est pas toxique pour les cellules HepG2 traitées avec une dose équivalente en fer que l'on trouve dans les milieux de culture standard sous forme de nitrate de fer nonahydrate Fe(NO₃)₃.9H₂O à la concentration de 0.1 mg/L.

Le contrôle négatif a été traité avec un milieu sans addition de Fer, mais la présence de sérum de veau fœtal a pu apporter suffisamment de fer pour permettre la croissance des cellules (donc le témoin n'est pas complètement négatif).

Le MTT a été pesé, été dissout à 5 mg/mL dans du milieu IMDM natif, filtré à 0,2µm sous hotte PSM et stocké à +4°C tout au long du protocole expérimental.

A chaque étape du test MTT, la solution à 5 mg/mL a été diluée à 0,5 mg/mL dans un milieu IMDM complet (IMDM + SVF + antibiotiques + pyruvate), mais sans Fer, puis incubé 3h à 37°C.

A chaque changement de milieu, le milieu usé a été prélevé et congelé à -20°C pour des dosages ultérieurs de transferrine et ferritine.

En conclusion, ce test démontre que les nanoclusters de fer de l'invention ne présentent aucune toxicité, quelle que soit leur concentration. Les cellules HepG2 cultivées dans un milieu sans fer puis avec ajout de fer présentent une excellente viabilité.

## Revendications

1. Nanoclusters de fer, **caractérisés en ce qu'**ils :
- sont recouverts à leur surface d'une couche mixte comprenant de l'histidine (His), des ions acétate (Ac) et des ions ascorbate (Asc),
- présentent une forme sphérique,
- présentent un diamètre hydrodynamique allant de 0,6 à 2,0 nm, et de préférence inférieur à 1,0 nm,
- présentent un diamètre de coeur métallique allant de 0,5 à 1,5 nm, et de préférence inférieur à 1,0 nm,
- présentent une stabilité dans le temps allant de 5 à 20 semaines lorsque les nanoclusters sont sous forme liquide et sont stockés à une température de 4°C,
- présentent une stabilité dans le temps d'au moins 12 mois, et de préférence de 12 à 18 mois lorsque les nanoclusters sont sous forme sèche et sont stockés à une température de 4°C et sous azote,
- présentent des propriétés spectrophotométriques, avec un épaulement sur le spectre UV-Visible à 300 ± 15 nm et un spectre de fluorescence avec des longueurs d'onde d'excitation de 364 ± 15 nm et des longueurs d'onde d'émission de 415 ± 15 nm,
lesdits nanoclusters de fer pouvant être désignés par la formule « FeNC@HisAcAsc ».

2. Nanoclusters de fer selon la revendication 1, **caractérisés en ce qu'**ils se présentent sous forme liquide ou sous forme sèche.

3. Nanoclusters de fer selon la revendication 1 ou la revendication 2, caractérisés en qu'ils présentent au moins une des caractéristiques suivantes :
- ils sont aptes à passer la barrière intestinale,
- ils présentent une bonne biodisponibilité,
- ils sont biocompatibles,
- ils sont biodégradables,
- ils sont lyophilisables,
- ils ne sont pas toxiques pour l'organisme humain,
- ils ne s'accumulent pas dans les organes tels que le foie, la rate, les reins ou les poumons.

4. Procédé de préparation de nanoclusters de fer selon l'une quelconque des revendications 1 à 3, **caractérisé en ce qu'**il comprend les étapes suivantes :
- réaction entre de l'acétate de fer (II) et de l'histidine afin d'obtenir un mélange d'acétate de fer et d'histidine, le ratio molaire histidine/acétate de fer (II) étant supérieur ou égal à 8, de préférence va de 8 à 200, et plus préférentiellement encore va de 80 à 200,
- réaction entre le mélange d'acétate de fer et d'histidine avec de l'acide ascorbique afin d'obtenir un mélange d'acétate de fer, d'histidine et d'acide ascorbique, le ratio molaire acide ascorbique/acétate de fer (II) étant supérieur ou égal à 12, de préférence va de 12 à 700, et plus préférentiellement encore va de 130 à 700,
- récupération des nanoclusters de fer.

5. Procédé de préparation selon la revendication 4, **caractérisé en ce qu'**il est effectué sous gaz inerte et **en ce que** :
- l'acétate de fer est sous forme de solution et l'histidine est sous forme de poudre,
- une solution d'acétate de fer et d'histidine est préparée par ajout d'histidine dans la solution d'acétate de fer,
- la solution d'acétate de fer et d'histidine est ajustée à une valeur de pH allant de 11 à 13, et de préférence est de 12,
- l'acide ascorbique est sous forme de poudre,
- une solution d'acétate de fer, d'histidine et d'acide ascorbique est préparée par ajout d'acide ascorbique à la solution d'acétate de fer et d'histidine dont le pH a été ajusté aux valeurs susmentionnées,
- la solution d'acétate de fer, d'histidine et d'acide ascorbique est agitée pendant 2h à 6h, et de préférence 4h, à une température allant de 35°C à 45°C, et de préférence 40°C,
- une solution comprenant des nanoclusters de fer est obtenue à l'issue de l'étape précédente d'agitation,
- la solution qui comprend les nanoclusters de fer est éventuellement dialysée afin d'obtenir une solution purifiée de nanoclusters de fer,
- la solution qui comprend les nanoclusters de fer, éventuellement dialysée, est éventuellement lyophilisée afin d'obtenir une forme sèche de nanoclusters de fer.

6. Procédé selon la revendication 5, caractérisé en qu'il comprend en outre au moins une caractéristique choisie parmi :
- le gaz inerte est de l'azote,
- la solution d'acétate de fer est préparée par ajout d'acétate de fer dans de l'eau ultra pure filtrée,
- la solution d'acétate de fer présente une concentration allant de 0,5 à 5,0 mM,
- la concentration d'histidine est supérieure à la concentration de la solution d'acétate de fer,
- le pH de la solution d'acétate de fer et d'histidine est ajusté à l'aide d'hydroxyde de sodium,
- la concentration d'acide ascorbique est égale à la concentration d'histidine,
- la solution qui comprend les nanoclusters de fer, éventuellement dialysée, présente une concentration en fer allant de 14 à 112 µg/mL,
- la solution qui comprend les nanoclusters de fer, éventuellement dialysée, est lyophilisée afin d'obtenir une forme sèche de nanoclusters de fer.

7. Procédé de préparation selon la revendication 4, **caractérisé en ce que** :
- l'acétate de fer est sous forme de poudre et l'histidine est sous forme de poudre,
- un mélange pulvérulent d'acétate de fer et d'histidine est obtenu par mélange de chacune des poudres d'acétate de fer et d'histidine,
- le mélange pulvérulent d'acétate de fer et d'histidine est broyé jusqu'à obtention d'un mélange pulvérulent homogène en couleur,
- le mélange pulvérulent homogène d'acétate de fer et d'histidine est placé dans un réacteur,
- l'acide ascorbique est sous forme de poudre,
- l'acide ascorbique est ajouté dans le réacteur comprenant le mélange pulvérulent homogène d'acétate de fer et d'histidine,
- le mélange pulvérulent d'acétate de fer, d'histidine et d'acide ascorbique ainsi obtenu est mis sous agitation, puis de l'eau est ajoutée goutte à goutte dans le réacteur, ladite eau étant de l'eau ultra pure filtrée,
- le réacteur est mis sous gaz inerte et à l'abri de la lumière,
- le mélange d'acétate de fer, d'histidine, d'acide ascorbique et d'eau est maintenu sous agitation dans le réacteur pendant 16 à 36h, et de préférence 24h,
- un mélange liquide qui comprend les nanoclusters de fer est obtenu à l'issue de l'étape précédente d'agitation,
- le mélange liquide qui comprend les nanoclusters de fer est éventuellement dialysé afin d'obtenir un mélange liquide purifié de nanoclusters de fer,
- le mélange liquide qui comprend les nanoclusters de fer, éventuellement dialysé, est éventuellement lyophilisé afin d'obtenir une forme sèche de nanoclusters de fer.

8. Procédé selon la revendication 7, **caractérisé en ce qu'**il comprend en outre au moins une caractéristique choisie parmi :
- la concentration d'histidine est supérieure à la concentration d'acétate de fer,
- la concentration d'acide ascorbique est égale à la concentration d'histidine,
- l'eau ajoutée dans le réacteur est de l'eau ultra pure filtrée,
- le gaz inerte est de l'azote,
- le mélange liquide qui comprend les nanoclusters de fer, éventuellement dialysé, présente une concentration en fer allant de 1500 à 15000 µg/mL,
- le mélange liquide qui comprend les nanoclusters de fer, éventuellement dialysé, est lyophilisé afin d'obtenir une forme sèche de nanoclusters de fer.

9. Procédé selon l'une quelconque des revendications 5 à 8, **caractérisé en ce que** la forme sèche des nanoclusters de fer est conservée sous azote, de préférence dans des flacons, et de préférence à 4°C, ladite poudre pouvant être conservée pendant une durée d'au moins 12 mois, et de préférence de 12 à 18 mois, sans altération de la stabilité des nanoclusters de fer.

10. Nanoclusters de fer tels que définis à l'une quelconque des revendications 1 à 3 ou tels qu'obtenus selon le procédé de l'une quelconque des revendications 4 à 9, pour une utilisation comme médicament.

11. Nanoclusters de fer tels que définis à l'une quelconque des revendications 1 à 3 ou tels qu'obtenus selon le procédé de l'une quelconque des revendications 4 à 9, pour une utilisation dans la prévention et/ou le traitement de pathologies générant un déficit en fer.

12. Nanoclusters de fer pour une utilisation selon la revendication 11, **caractérisés en ce que** la pathologie générant un déficit en fer est une anémie ferriprive.

13. Nanoclusters de fer tels que définis à l'une quelconque des revendications 1 à 3 ou tels qu'obtenus selon le procédé de l'une quelconque des revendications 4 à 9, pour une utilisation pour lutter contre les carences en fer.

14. Composition **caractérisée en ce qu'**elle comprend des nanoclusters de fer tels que définis à l'une quelconque des revendications 1 à 3 ou tels qu'obtenus selon le procédé de l'une quelconque des revendications 4 à 9.

15. Composition selon la revendication 14, **caractérisée en ce qu'**il s'agit d'un médicament, d'un complément alimentaire ou d'une composition alimentaire.

16. Composition selon la revendication 14 ou 15, **caractérisée en ce qu'**elle se trouve sous une forme adaptée pour une administration par voie orale.

## Patentansprüche

1. Eisen-Nanocluster, **dadurch gekennzeichnet, dass** sie:
- an ihrer Oberfläche mit einer Mischschicht bedeckt sind, die Histidin (His), Acetat-Ionen (Ac) und Ascorbat-Ionen (Asc) umfasst,
- eine kugelförmige Gestalt aufweisen,
- einen hydrodynamischen Durchmesser im Bereich von 0,6 bis 2,0 nm und bevorzugt unter 1,0 nm aufweisen,
- einen Durchmesser des Metallkerns im Bereich von 0,5 bis 1,5 nm und bevorzugt unter 1,0 nm aufweisen,
- eine zeitliche Stabilität von 5 bis 20 Wochen aufweisen, wenn sich die Nanocluster in flüssiger Form befinden und bei einer Temperatur von 4 °C gelagert werden,
- eine zeitliche Stabilität von wenigstens 12 Monaten und bevorzugt von 12 bis 18 Monaten aufweisen, wenn die Nanocluster in trockener Form vorliegen und bei einer Temperatur von 4 °C unter Stickstoff gelagert werden,
- spektralphotometrische Eigenschaften aufweisen, mit einer Schulter im UV/Vis-Spektrum bei 300 ± 15 nm und einem Fluoreszenzspektrum mit Anregungswellenlängen von 364 ± 15 nm und Emissionswellenlängen von 415 ± 15 nm,
wobei die Eisen-Nanocluster durch die Formel "FeNC@HisAcAsc" bezeichnet werden können.

2. Eisen-Nanocluster nach Anspruch 1, **dadurch gekennzeichnet, dass** sie in flüssiger oder trockener Form vorliegen.

3. Eisen-Nanocluster nach Anspruch 1 oder Anspruch 2, **dadurch gekennzeichnet, dass** sie wenigstens eines der folgenden Merkmale aufweisen:
- sie sind in der Lage, die Darmbarriere zu passieren,
- sie weisen eine gute Bioverfügbarkeit auf,
- sie sind biokompatibel,
- sie sind biologisch abbaubar,
- sie sind lyophilisierbar,
- sie sind für den menschlichen Organismus nicht toxisch,
- sie reichern sich nicht in Organen wie Leber, Milz, Nieren oder Lunge an.

4. Verfahren zur Herstellung von Eisen-Nanoclustern nach einem der Ansprüche 1 bis 3, **dadurch gekennzeichnet, dass** es die folgenden Schritte umfasst:
- Reaktion von Eisen(II)-acetat mit Histidin, um ein Gemisch aus Eisenacetat und Histidin zu erhalten, wobei das Molverhältnis Histidin/ Eisen(II)-acetat größer oder gleich 8 ist, bevorzugt im Bereich von 8 bis 200 liegt und stärker bevorzugt im Bereich von 80 bis 200 liegt,
- Reaktion des Gemisches aus Eisenacetat und Histidin mit Ascorbinsäure, um ein Gemisch aus Eisenacetat, Histidin und Ascorbinsäure zu erhalten, wobei das Molverhältnis Ascorbinsäure/ Eisen(II)-acetat größer oder gleich 12 ist, bevorzugt im Bereich von 12 bis 700 liegt und noch stärker bevorzugt im Bereich von 130 bis 700 liegt,
- Gewinnung der Eisen-Nanocluster.

5. Verfahren zur Herstellung nach Anspruch 4, **dadurch gekennzeichnet, dass** es unter Inertgas durchgeführt wird und dass:
- das Eisenacetat in Form einer Lösung und das Histidin in Pulverform vorliegen,
- eine Lösung aus Eisenacetat und Histidin durch Zugabe von Histidin zur Eisenacetatlösung hergestellt wird,
- die Lösung aus Eisenacetat und Histidin auf einen pH-Wert im Bereich von 11 bis 13 eingestellt wird, und bevorzugt 12 ist,
- Ascorbinsäure in Pulverform vorliegt,
- eine Lösung aus Eisenacetat, Histidin und Ascorbinsäure hergestellt wird, indem Ascorbinsäure zu der Lösung aus Eisenacetat und Histidin hinzugefügt wird, deren pH-Wert auf die oben genannten Werte eingestellt wurde,
- die Lösung aus Eisenacetat, Histidin und Ascorbinsäure 2 bis 6 Stunden, bevorzugt 4 Stunden, bei einer Temperatur von 35 °C bis 45 °C, bevorzugt 40 °C, gerührt wird,
- nach Abschluss des vorhergehenden Rührschritts eine Lösung erhalten wird, die Eisen-Nanocluster umfasst,
- die Lösung, die Eisen-Nanocluster umfasst, gegebenenfalls dialysiert wird, um eine gereinigte Lösung von Eisen-Nanoclustern zu erhalten,
- die Lösung, die die Eisen-Nanocluster enthält und gegebenenfalls dialysiert wurde, gegebenenfalls lyophilisiert wird, um eine trockene Form von Eisen-Nanoclustern zu erhalten.

6. Verfahren nach Anspruch 5, **dadurch gekennzeichnet, dass** es ferner wenigstens ein Merkmal umfasst, gewählt aus:
- das Inertgas ist Stickstoff,
- die Eisenacetatlösung wird durch Zugabe von Eisenacetat zu gefiltertem ultrareinem Wasser hergestellt,
- die Eisenacetatlösung weist eine Konzentration im Bereich von 0,5 bis 5,0 mM auf,
- die Histidinkonzentration ist höher als die Konzentration der Eisenacetatlösung,
- der pH-Wert der Eisenacetat- und Histidinlösung wird mit Natriumhydroxid eingestellt,
- die Ascorbinsäurekonzentration entspricht der Histidinkonzentration,
- die Lösung, die die Eisen-Nanocluster umfasst, gegebenenfalls dialysiert, weist eine Eisenkonzentration im Bereich von 14 bis 112 µg/ml auf,
- die Lösung, die die Eisen-Nanocluster umfasst, gegebenenfalls dialysiert, wird lyophilisiert, um eine trockene Form von Eisen-Nanoclustern zu erhalten.

7. Verfahren nach Anspruch 4, **dadurch gekennzeichnet, dass**:
- das Eisenacetat in Pulverform und das Histidin in Pulverform vorliegen,
- eine pulverförmige Mischung aus Eisenacetat und Histidin durch Mischen der jeweiligen Pulver aus Eisenacetat und Histidin erhalten wird,
- die pulverförmige Mischung aus Eisenacetat und Histidin gemahlen wird, bis eine farblich homogene pulverförmige Mischung erhalten ist,
- die homogene pulverförmige Mischung aus Eisenacetat und Histidin in einen Reaktor gegeben wird,
- Ascorbinsäure in Pulverform vorliegt,
- die Ascorbinsäure in den Reaktor gegeben wird, der die homogene pulverförmige Mischung aus Eisenacetat und Histidin umfasst,
- die so erhaltene pulverförmige Mischung aus Eisenacetat, Histidin und Ascorbinsäure gerührt wird, anschließend tropfenweise Wasser in den Reaktor gegeben wird, wobei es sich bei diesem Wasser um gefiltertes ultrareines Wasser handelt,
- der Reaktor unter Inertgas gestellt und vor Licht geschützt wird,
- die Mischung aus Eisenacetat, Histidin, Ascorbinsäure und Wasser im Reaktor 16 bis 36 Stunden, und bevorzugt 24 Stunden, unter Rühren gehalten wird,
- nach Abschluss des vorhergehenden Rührschritts eine flüssige Mischung erhalten wird, die die Eisen-Nanocluster umfasst,
- die flüssige Mischung, die die Eisen-Nanocluster umfasst, gegebenenfalls dialysiert wird, um eine gereinigte flüssige Mischung aus Eisen-Nanoclustern zu erhalten,
- die flüssige Mischung, die die Eisen-Nanocluster enthält, gegebenenfalls dialysiert, gegebenenfalls lyophilisiert wird, um eine trockene Form von Eisen-Nanoclustern zu erhalten.

8. Verfahren nach Anspruch 7, **dadurch gekennzeichnet, dass** es ferner wenigstens ein Merkmal umfasst, gewählt aus:
- die Histidin-Konzentration ist höher als die Eisenacetat-Konzentration,
- die Ascorbinsäure-Konzentration entspricht der Histidin-Konzentration,
- das in den Reaktor zugegebene Wasser ist gefiltertes ultrareines Wasser,
- das Inertgas ist Stickstoff,
- die flüssige Mischung, die die Eisen-Nanocluster umfasst, gegebenenfalls dialysiert, weist eine Eisenkonzentration im Bereich von 1500 bis 15000 µg/ml auf,
- die flüssige Mischung, die die Eisen-Nanocluster umfasst, gegebenenfalls dialysiert, wird lyophilisiert, um eine trockene Form von Eisen-Nanoclustern zu erhalten.

9. Verfahren nach einem der Ansprüche 5 bis 8, **dadurch gekennzeichnet, dass** die trockene Form der Eisen-Nanocluster unter Stickstoff, bevorzugt in Fläschchen, und bevorzugt bei 4 °C aufbewahrt wird, wobei das Pulver über einen Zeitraum von wenigstens 12 Monaten, bevorzugt von 12 bis 18 Monaten, ohne Beeinträchtigung der Stabilität der Eisen-Nanocluster gelagert werden kann.

10. Eisen-Nanocluster, wie in einem der Ansprüche 1 bis 3 definiert oder wie nach dem Verfahren eines der Ansprüche 4 bis 9 erhalten, zur Verwendung als Arzneimittel.

11. Eisen-Nanocluster, wie in einem der Ansprüche 1 bis 3 definiert oder wie nach dem Verfahren eines der Ansprüche 4 bis 9 erhalten, zur Verwendung bei der Vorbeugung und/oder Behandlung von Erkrankungen, die einen Eisenmangel verursachen.

12. Eisen-Nanocluster zur Verwendung nach Anspruch 11, **dadurch gekennzeichnet, dass** die zu einem Eisenmangel führende Erkrankung eine Eisenmangelanämie ist.

13. Eisen-Nanocluster, wie in einem der Ansprüche 1 bis 3 definiert oder wie nach dem Verfahren eines der Ansprüche 4 bis 9 erhalten, zur Verwendung bei der Bekämpfung von Eisenmangel.

14. Zusammensetzung, **dadurch gekennzeichnet, dass** sie Eisen-Nanocluster umfasst, wie in einem der Ansprüche 1 bis 3 definiert oder wie nach dem Verfahren eines der Ansprüche 4 bis 9 erhalten.

15. Zusammensetzung nach Anspruch 14, **dadurch gekennzeichnet, dass** es sich um ein Arzneimittel, ein Nahrungsergänzungsmittel oder eine Lebensmittelzusammensetzung handelt.

16. Zusammensetzung nach Anspruch 14 oder 15, **dadurch gekennzeichnet, dass** sie in einer für eine orale Verabreichung geeigneten Form vorliegt.

## Claims

1. Iron nanoclusters, **characterized in that** they:
- are covered on their surface with a mixed layer comprising histidine (His), acetate ions (Ac), and ascorbate ions (Asc),
- have a spherical shape,
- have a hydrodynamic diameter ranging from 0.6 to 2.0 nm, and preferably of less than 1.0 nm,
- have a metal core diameter ranging from 0.5 to 1.5 nm, and preferably of less than 1.0 nm,
- exhibit a stability duration ranging from 5 to 20 weeks when the nanoclusters are in liquid form and are stored at a temperature of 4°C,
- exhibit a stability duration of at least 12 months, and preferably from 12 to 18 months, when the nanoclusters are in dry form and are stored at a temperature of 4°C and under nitrogen,
- exhibit spectrophotometric properties, with a shoulder on the UV-Visible spectrum at 300 ± 15 nm and a fluorescence spectrum with excitation wavelengths of 364 ± 15 nm and emission wavelengths of 415 ± 15 nm,
it being possible to refer to said iron nanoclusters by using the formula "FeNC@HisAcAsc".

2. Iron nanoclusters according to claim 1, **characterized in that** they are in liquid form or dry form.

3. Iron nanoclusters according to claim 1 or claim 2, **characterized in that** they exhibit at least one of the following characteristics:
- they are able to cross the intestinal barrier,
- they exhibit good bioavailability,
- they are biocompatible,
- they are biodegradable,
- they are able to be freeze-dried,
- they are non-toxic to the human body,
- they do not accumulate in organs such as the liver, spleen, kidneys, or lungs.

4. A method of preparing iron nanoclusters according to any one of claims 1 to 3, **characterized in that** it comprises the following steps:
- reaction of iron (II) acetate with histidine in order to obtain a mixture of iron acetate and histidine, the molar ratio of histidine/iron (II) acetate being greater than or equal to 8, preferably ranging from 8 to 200, and more preferably ranging from 80 to 200,
- reaction of the mixture of iron acetate and histidine with ascorbic acid in order to obtain a mixture of iron acetate, histidine, and ascorbic acid, the molar ratio of ascorbic acid/iron (II) acetate being greater than or equal to 12, preferably ranging from 12 to 700, and more preferably ranging from 130 to 700,
- collecting the iron nanoclusters.

5. Method of preparation according to claim 4, **characterized in that** it is carried out under inert gas and **in that**:
- the iron acetate is in solution form and the histidine is in powder form,
- a solution of iron acetate and histidine is prepared by adding histidine to the iron acetate solution,
- the solution of iron acetate and histidine is adjusted to a pH value ranging from 11 to 13, and preferably 12,
- the ascorbic acid is in powder form,
- a solution of iron acetate, histidine, and ascorbic acid is prepared by adding ascorbic acid to the solution of iron acetate and histidine for which the pH has been adjusted to the aforementioned values,
- the solution of iron acetate, histidine, and ascorbic acid is stirred for 2 to 6 hours, and preferably 4 hours, at a temperature ranging from 35°C to 45°C, and preferably 40°C,
- a solution comprising iron nanoclusters is obtained at the end of the previous stirring step,
- the solution comprising iron nanoclusters is optionally dialyzed to obtain a purified solution of iron nanoclusters,
- the solution comprising iron nanoclusters, optionally dialyzed, is optionally freeze-dried to obtain a dry form of iron nanoclusters.

6. Method according to claim 5, **characterized in that** it further comprises at least one characteristic selected from the following:
- the inert gas is nitrogen,
- the iron acetate solution is prepared by adding iron acetate to filtered ultrapure water,
- the iron acetate solution has a concentration ranging from 0.5 to 5.0 mM,
- the histidine concentration is higher than the concentration of the iron acetate solution,
- the pH of the solution of iron acetate and histidine is adjusted using sodium hydroxide,
- the ascorbic acid concentration is equal to the histidine concentration,
- the solution comprising iron nanoclusters, optionally dialyzed, has an iron concentration ranging from 14 to 112 µg/mL,
- the solution comprising iron nanoclusters, optionally dialyzed, is freeze-dried to obtain a dry form of iron nanoclusters.

7. Method of preparation according to claim 4, **characterized in that**:
- the iron acetate is in powder form and the histidine is in powder form,
- a powder mixture of iron acetate and histidine is obtained by mixing together each of the iron acetate and histidine powders,
- the powder mixture of iron acetate and histidine is ground until a powder mixture of homogeneous color is obtained,
- the homogeneous powder mixture of iron acetate and histidine is placed in a reactor,
- the ascorbic acid is in powder form,
- the ascorbic acid is added to the reactor comprising the homogeneous powder mixture of iron acetate and histidine,
- the thus obtained powder mixture of iron acetate, histidine, and ascorbic acid is stirred, then water is added dropwise into the reactor, said water being filtered ultrapure water,
- the reactor is placed under inert gas and protected from light,
- the mixture of iron acetate, histidine, ascorbic acid, and water is left to stir in the reactor for 16 to 36 hours, and preferably 24 hours,
- a liquid mixture comprising iron nanoclusters is obtained at the end of the previous stirring step,
- the liquid mixture comprising iron nanoclusters is optionally dialyzed to obtain a purified liquid mixture of iron nanoclusters,
- the liquid mixture comprising iron nanoclusters, optionally dialyzed, is optionally freeze-dried to obtain a dry form of iron nanoclusters.

8. Method according to claim 7, **characterized in that** it further comprises at least one characteristic selected from the following:
- the histidine concentration is greater than the iron acetate concentration,
- the ascorbic acid concentration is equal to the histidine concentration,
- the water added to the reactor is filtered ultrapure water,
- the inert gas is nitrogen,
- the liquid mixture comprising iron nanoclusters, optionally dialyzed, has an iron concentration ranging from 1500 to 15000 µg/mL,
- the liquid mixture comprising iron nanoclusters, optionally dialyzed, is freeze-dried to obtain a dry form of iron nanoclusters.

9. Method according to any one of claims 5 to 8, **characterized in that** the dry form of the iron nanoclusters is stored under nitrogen, preferably in vials, and preferably at 4°C, it being possible to store said powder for a period of at least 12 months, and preferably from 12 to 18 months, with no change in the stability of the iron nanoclusters.

10. Iron nanoclusters as defined in any one of claims 1 to 3 or as obtained according to the method of any one of claims 4 to 9, for use as medication.

11. Iron nanoclusters as defined in any one of claims 1 to 3 or as obtained according to the method of any one of claims 4 to 9, for use in the prevention and/or treatment of diseases causing iron deficiency.

12. Iron nanoclusters for use according to claim 11, **characterized in that** the disease causing an iron deficiency is iron deficiency anemia.

13. Iron nanoclusters as defined in any one of claims 1 to 3 or as obtained according to the method of any one of claims 4 to 9, for use in combating iron deficiencies.

14. Composition **characterized in that** it comprises iron nanoclusters as defined in any one of claims 1 to 3 or as obtained according to the method of any one of claims 4 to 9.

15. Composition according to claim 14, **characterized in that** it is a medication, a dietary supplement, or a food composition.

16. Composition according to claim 14 or 15, **characterized in that** it is in a form suitable for oral administration.
